# EUROPEAN PATENT APPLICATION

(11) **EP 2 281 623 A1**
(43) Date of publication of application: **09.02.2011**
(21) Application number: 09176524.8
(22) Date of filing: 19.11.2009
(51) Int. Cl.: B01D 61/02, C07C 231/24, C07C 237/46

(54) **Processing crude iodixanol mixture by nanofiltration**

(30) Priority: 21.07.2009 US 227101 P
(71) Applicant: GE Healthcare AS, 0401 Oslo (NO)
(72) Inventor: Homestad, Ole, Magne, 4521 Spangereid (NO); Ingvoldstad, Odd, Einar, 4521 Spangereid (NO)
(74) Representative: Wulff, Marianne Weiby

(57) **Abstract**

This invention relates generally to industrial preparation of iodixanol (1,3-bis(acetamido)-N,N'-bis[3,5-bis(2,3-dihydroxypropylaminocarbonyl)-2,4,6-triiodophenyl]-2-hydroxypropane), a non-ionic X-ray contrasting agent. It further relates to a method for preparing a crude mixture of the dimcrisation reaction from 5-acctamido-N,N'-bis(2,3-dihydroxypropyl)-2,4,6-triiodoisophthalamidc ("Compound A") to iodixanol for the crystallization of iodixanol. In particular, it relates to an industrial procedure of simultaneously reducing the salt content and the alcoholic dimerisation solvent using a nanofiltration system prior to the crystallization of iodixanol.

## Description

### TECHNICAL FIELD

This invention relates generally to industrial preparation of iodixanol (1,3-bis(acetamido)-N,N'-bis[3,5-bis(2,3-dihydroxypropylaminocarbonyl)-2,4,6-triiodophenyl]-2-hydroxypropane), a non-ionic X-ray contrasting agent. It further relates to an improved method in the purification of iodixanol. In particular, it relates to reducing the salt content and the alcoholic solvent content using a nanofiltration system prior to the crystallization of iodixanol.

### BACKGROUND OF THE INVENTION

Iodixanol is the non-proprietary name of the chemical drug substance, 1,3-bis(acetamido)-N,N'-bis[3,5-bis(2,3-dihydroxypropylaminocarbonyl)-2,4,6-triiodophenyl]-2-hydroxypropane). It is a one of the most used agents in diagnostic X-ray procedures and marketed under the trade name Visipaque®. It is produced in large quantities by GE Healthcare in Lindesnes, Norway. The manufacture of iodixanol involves the production of the chemical drug substance (referred to as primary production) followed by formulation into the drug product (referred to as secondary production). Primary production of iodixanol involves a multistep chemical synthesis and a thorough purification process. For a commercial drug product, it is important for the primary production to be efficient and economical and to provide a drug substance fulfilling the regulatory specifications, such as those mandated by US Pharmacopeia. In addition, the cost and efficiency of the secondary production depend on the synthesis and purification processes in the primary production. It is therefore critical to optimize each process in the primary production of iodixanol.

The industrial synthesis of iodixanol involves dimerisation of intermediate 5-acetamido-N,N'-bis(2,3-dihydroxypropyl)-2,4,6-triiodoisophthalamide ("Compound A") as the final synthetic step. *See* Scheme 1 below and U.S. Patent No. 6,974,882.

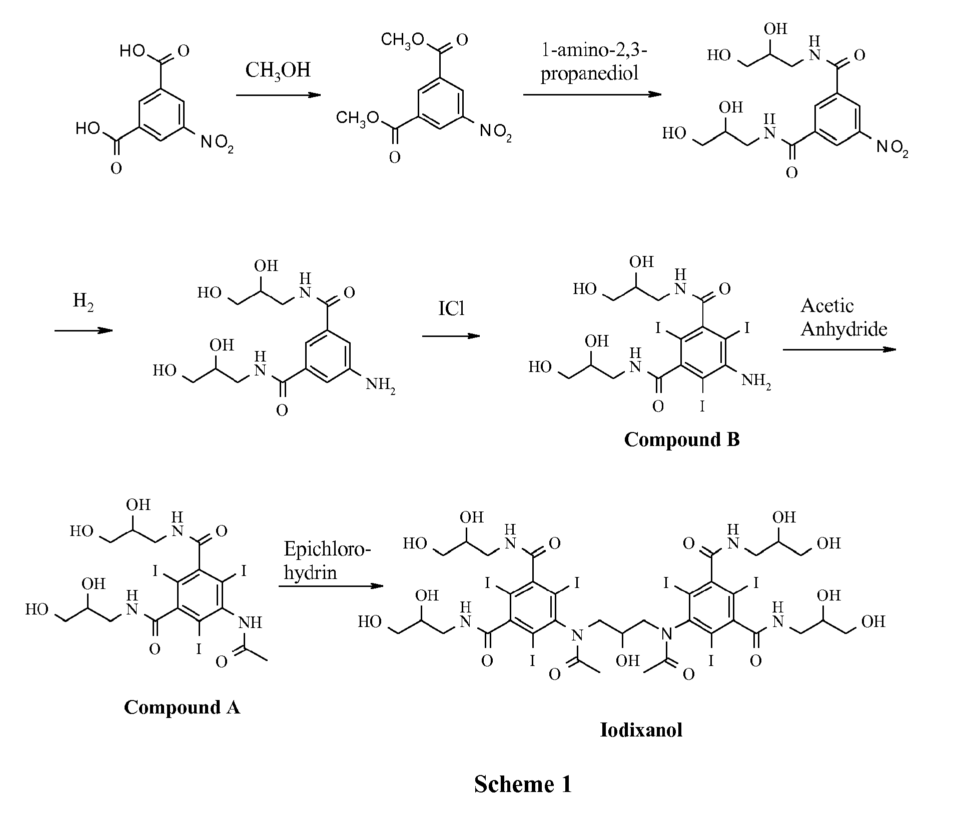

The reaction mixture after the dimerisation of Compound A to iodixanol contains a considerable amount of salt, mainly in the form of NaCl. The sources of chloride are epichlorohydrin, which is added to the reaction in an amount of about 0.33 mole/mole Compound A, and hydrochloric acid, which is used to adjust the pH before epichlorohydrin addition and to precipitate unreacted Compound A after the reaction. The source of sodium cations is the NaOH used to dissolve Compound A in the reaction solvent. More than one equivalent of NaOH is required to dissolve Compound A in the anionic form. In addition, the dimerisation reaction mixture contains a large amount of alcoholic solvent, such as 2-methoxyethanol, methanol, propylene glycol, or 1-methoxy-2-propanol. It is thus desirable to devise a cost effective procedure to work up the crude dimerisation reaction mixture for subsequent purification steps.

### SUMMARY OF THE INVENTION

The present invention is directed to an industrial procedure for processing a crude reaction mixture following the dimerisation of Compound A to iodixanol in an alcoholic solvent. Such reaction mixture typically contains more than about 12 weight % of salt content relative to iodixanol content. The instant process comprises a series of sequential steps to prime the crude reaction mixture prior to the crystallization of iodixanol. These steps include feeding the dimerisation reaction mixture containing Compound A and iodixanol in an alcoholic solvent into a filtration system, passing the filtrate through a nanofiltration system wherein water is added to the retentate side; and recovering the retentate wherein the salt content is less than about 0.6 weight % to iodixanol content and is substantially free of the alcoholic solvent used in the dimerisation reaction. The alcoholic dimerisation solvent may include 2-methoxyethanol, methanol, propylene glycol, and 1-methoxy-propanol.

### DETAILED DESCRIPTION OF THE INVENTION

In the final step of the primary production process, iodixanol has to be purified. A common method for purification is by crystallisation. In order to crystallise iodixanol in accordance with the regulatory purity requirement, we have found that the salt content prior to the crystallisation process should be less than about 0.6 w/w % relative to iodixanol.

It has also been found that the presence of alcoholic solvent used in the dimerisation has a detrimental effect on the crystallization yield of iodixanol. We have found that even a small amount of alcoholic solvent increases the solubility of iodixanol in the crystallisation mixture and hence reduces the crystallisation yield considerably.

To achieve the low salt and low alcoholic solvent levels, many attempts have been to devise a process to effectively and efficiently reduce large salt content following the dimerisation reaction. It has been found that a combination of concentration and diafiltration with an aqueous solvent can be successfully used to simultaneously reduce the salt content and the alcoholic solvent content to the desired levels in a cost-effective manner. Only a minimal amount of iodixanol is lost during the instant process.

The above process addresses the specific need of the current iodixanol synthesis methodology, where a large amount of salt is generated as a result of the dimerisation reagent and the quenching reagent selected. The instant process also represents a significant improvement over the alternative of employing ion exchange resins. For example, two ion exchange resins, one anionic and one cationic, are needed to remove the significant amount of salts present following the dimerisation reaction. The requirement of two resins, both in large quantities, causes significant loss of Compound A and iodixanol. In addition, the operation of large scale purifications using ion exchange resins (such as separation time, energy consumption, cost of resin regeneration and replacement, and equipment requirement) is lengthy, complex, and expensive.

In addition, the instant process is superior to the alternative of using evaporation to reduce alcoholic solvent content. For example, distillation or any other form of evaporation is both energy consuming and potentially thermally stressing to the iodixanol product.

The invention is illustrated further by the following examples that are not to be construed as limiting the invention in scope to the specific procedures described in them.

### EXAMPLES

### EXAMPLE 1

Compound A (600 kg) is reacted with epichlorohydrin (0.33 eq) in an alcoholic solvent in the presence of sodium hydroxide at a pH of about 11.9 at 15°C. About 55 % conversion to iodixanol is obtained. Most of unreacted Compound A is precipitated from the reaction mixture by addition of hydrochloric acid followed by filtration. The aqueous filtrate contains about 340 kg iodixanol, 100 kg Compound A and 20 kg iohexol. The pH is measured to about 4-6. The NaCl content is about 12-14 w/w % relative to iodixanol. The solution is then subjected to nanofiltration. Water is added continuously to facilitate diafiltration followed by volume reduction. A final salt concentration of about 0.60 w/w % relative to iodixanol (2.0 kg NaCl in 340 kg iodixanol) is obtained. The alcoholic solvent is removed through the membrane in the diafiltration process, resulting in an aqueous process solution ready for the next process step.

All patents, journal articles, publications and other documents discussed and/or cited above are hereby incorporated by reference.

## Claims

1. An industrial process for priming a crude reaction mixture of dimerisation of 5-acetamido-N,N'-bis(2,3-dihydroxypropyl)-2,4,6-triiodoisophthalamide ("Compound A") to iodixanol before cystallisation of iodixanol, comprising the sequential steps of
(1) feeding the dimerisation reaction mixture containing Compound A and iodixanol in an alcoholic solvent into a filtration system;
(2) passing the filtrate of (1) through a nanofiltration system wherein water is added to the retentate side; and
(3) recovering the retentate wherein the salt content is less than about 0.6 weight % to iodixanol content and is substantially free of the alcoholic solvent used in the dimerisation reaction.
